# EUROPEAN PATENT SPECIFICATION

(11) **EP 1 066 040 B1**
(45) Date of publication and mention of the grant of the patent: **19.02.2003**
(21) Application number: 99937874.8
(22) Date of filing: 03.03.1999
(51) Int. Cl.: A61K 31/545

(54) **PHARMACEUTICAL COMPOSITION CONTAINING CEFUROXIME AXETIL STABLE FOR MOISTURE ABSORPTION**
FEUCHTIGKEITSSTABILE CEFUROXIM AXETIL ENTHALTENDE PHARMAZEUTISCHE ZUSAMMENSTELLUNGEN
COMPOSITION PHARMACEUTIQUE CONTENANT DU CEFUROXIME AXETIL STABLE A L'ABSORPTION D'HUMIDITE

(30) Priority: 03.03.1998 KR 9806866; 18.08.1998 KR 9833463
(43) Date of publication of application: 10.01.2001
(73) Proprietor: Daewoong Pharmaceutical Co., Ltd., Sungnam-city, Kyunggi-do 462-120 (KR); Daewoong Chemical, Co., Ltd., Hwasung-gun, Kyunggi-do 445-920 (KR)
(72) Inventor: CHUNG, J. K., Moogoonghwa Apartment, Kyunggi-do 435-050 (KR); KIM, H. H., Sansung Apartment, Sungnam-shi,, Kyunggi-do 462-121 (KR); LIM, S. H., Shinheung Joogong Apartment, Sungnam-shi, Kyunggi-do 461-162 (KR); PARK, Jeong, Hwa, Seoul 135-280 (KR); PARK, J. W., Hangang Woosung Apartment 101-1511, Seoul 143-302 (KR)
(74) Representative: Dost, Wolfgang, Dr.rer.nat., Dipl.-Chem.
(86) International application number: KR9900095
(87) International publication number: WO99044614

(56) References cited:
- EP-A2- 0 280 571
- GB-A- 2 127 401
- GB-A- 2 181 052

## Description

The present invention relates to a pharmaceutical composition containing cefuroxime axetil. More specifically, the present invention relates to a pharmaceutical composition which comprises (i) amorphous cefuroxime axetil as an active ingredient, and (ii) silicon dioxide or its hydrate as a micro-environmental pH adjustor around cefuroxime axetil and an anti-gelling agent for cefuroxime axetil, wherein the weight ratio of cefuroxime axetil and silicon dioxide or its hydrate is 1:0.1 to 1:1.

Cefuroxime is an antibiotic showing a broad anti-bacterial activity spectrum against gram-positive and gram-negative bacteria as disclosed in UK Patent No. 1,453,049. Cefuroxime and its salts have been generally administered as an injectable preparation because they are insufficiently absorbed from the gastrointestinal tract.

As an effort to solve the problem involved with the administration of cefuroxime into the body, cefuroxime axetil was developed as a prodrug which can be administered via oral route. Cefuroxime axetil is an ester derivative of ceftiroxime in which 4-carboxylic group of cefuroxime is esterified with 1-acetoxyethyl group. As described in UK Patent No. 1,571,683, if 1-acetoxyethyl ester group exists in cefuroxime, the ester derivative can be administered via oral route because the lipophilicity of cefuroxime is increased and thus, the absorption of the drug from the gastrointestinal tract is increased. After being administered by oral route, cefuroxime axetil is rapidly hydrolyzed by non-specific esterases present in the intestinal mucosa and blood within the body to exert the pharmacological effect as a drug, cefuroxime. It is especially advantageous to use cefuroxime axetil in an amorphous form as described in UK Patents No. 2,127,401 and No. 2, 181, 052.

However, since cefuroxime axetil has a strong bitter taste which cannot be easily masked, an appropriate mean to mask the bitter taste is required in order to formulate it for oral administration. When formulating an active ingredient with a bitter taste into tablets, the method for masking the bitter taste generally used in the art is to coat tablet with a film. However, as described in Korean Patent No. 73572, there are drawbacks that if cefuroxime axetil contacts aqueous medium, a mass of gelatin shape is produced and, when it is administered in the form of a film coated tablet, cefuroxime axetil contained in the tablet core is gelled even if gastric juice is relatively slowly penetrated into the tablet core through the film, thereby resulting in insufficient absorption. Due to such a gel formation, the tablet core is insufficiently disintegrated and thus, the dissolution of the cefuroxime axetil is lowered. Therefore, it was impossible to provide the desired pharmacological effect because the absorption of the drug into the gastrointestinal tract is considerably decreased.

In order to solve these drawbacks, a cefuroxime axetil tablet comprising a film which can be rapidly ruptured in 0.07 M of aqueous hydrochloric acid solution and a tablet core which is disintegrated immediately after the film is ruptured was developed. [See, Korean Patent No.73572]. This method can be said as the one which the problem of gel formation is overcome and high bioavailability is maintained by a film coated tablet in which, upon contact with gastrointestinal juice, the film coating ruptures very rapidly and the core then immediately disintegrates, thereby allowing dispersion and dissolution of cefuroxime axetil in the gastrointestinal tract before gel formation.

However, the thin film coated with the water soluble film forming material cannot completely block the effect by outer environment. That is, moisture in air is permeated into the core of tablets upon storage and as a result, the cefuroxime axetil is gelled within the core of the tablet giving poor dissolution. Therefore, there is a serious problem that dissolution rate of the core is decreased from the gastro-intestinal tract and the bioavailability of cefuroxime axetil is also decreased.

The foregoing can be found from the fact that if cefuroxime axetil tablet is stored in a brown glass bottle in an opened state under the accelerated condition of 40 °C, and 75% of relative humidity, the moisture content is increased by 2 - 3 % even after the time lapse of one month and it takes more than 60 minutes for the tablet to be disintegrated [See, a Japanese journal of "Antibiotics & Chemotherapy", Vol 7, No. 11, 1991]. Further, the present inventors have confirmed that an undissolved mass was frequently remained even after 45 minutes when subjecting the tablet prepared by the above method to the dissolution test method as described in the monograph of the cefuroxime axetil tablets of Vol. 23, page 316 of the U.S. Pharmacopeia. Due to this mass, the drug is not completely dissolved and the bioavailability is decreased. Therefore, the method whereby the film is rapidly ruptured and the tablet core is immediately disintegrated using the water soluble film forming material as disclosed in Korean Patent No. 73572 may be effective in the prevention of gelation of the cefuroxime axetil by gastric juice and in increasing the dissolution rate upon administration to the patient, thereby improving the bioavailability in a very short period of storage. However, the preparation according to said method still has a problem that it cannot completely block the absorption of moisture into the core upon long term storage and thus rather may cause gelation of the active ingredient within the core of the tablet upon storage.

As an effort to prevent the gelation of the cefuroxime axetil particles, Korean Patent Publication No. 95-9097 discloses a method for preparing a cefuroxime axetil-containing composition in a finely divided particle form coated with lipid which is water-insoluble, masks bitter taste and is dispersed readily upon contact with gastric juice.

However, the above method has drawbacks that it must use cefuroxime axetil having the particle size less than 250 µm and should employ a complicated coating process in order to assure the prevention of the absorption of moisture. Therefore, this method is also inconvenient for preparing solid formulation of cefuroxime axetil for oral administration.

### DISCLOSURE OF THE INVENTION

It is therefore an object of the present invention to provide a pharmaceutical composition in which the above problems were eliminated or remarkably improved.

Another object of the present invention is to provide a pharmaceutical composition which comprises (i) amorphous cefuroxime axetil as an active ingredient, and (ii) silicon dioxide or its hydrate as a micro-environmental pH adjustor around cefuroxime axetil and an anti-gelling agent for cefuroxime axetil, wherein the weight ratio of cefuroxime and silicon dioxide or its hydrate is 1:0.1 to 1:1.

Still another object of the present invention is to provide a pharmaceutical composition in an oral formulation such as tablet, capsule, granule, powder and dry syrup.

Further objects and advantages of the invention will become apparent through reading the remainder of the specification.

The foregoing has outlined some of the more pertinent objects of the present invention. These objects should be construed to be merely illustrative of some of the more pertinent features of the invention. Many other beneficial results can be obtained by applying the disclosed invention in a different manner or by modifying the invention within the scope of the disclosure. Accordingly, other objects and a more thorough understanding of the invention may be found by referring to the detailed description of the preferred embodiment in addition to the scope of the invention defined by the claims.

The present inventors have conducted an extensive research for many years in order to develop a stable oral preparation wherein the gel formation of cefuroxime axetil which may occur upon storage can be effectively prevented by employing a simple process that does not require the complicated coating process as mentioned in connection with the above Korean patent. As a result, the inventors have surprisingly discovered that when a composition, in which silicon dioxide or its hydrate serving as a micro-environmental pH adjustor near cefuroxime axetil and an anti-gelling agent for cefuroxime axetil is mixed with cefuroxime axetil, is provided as an oral formulation, silicon dioxide or its hydrate completely prevented the degradation or gel formation of cefuroxime axetil due to absorption of moisture in air and the gel formation of cefuroxime axetil by the permeation of the gastric juice into the tablet core upon administration and make it possible to increase the dissolution rate upon administration to the patient, thereby improving the bioavailability, and have completed the present invention.

### BRIEF DESCRIPTION OF DRAWINGS

Fig. 1 is a graph according to the result of Experiment 1 showing the dissolution rate of cefuroxime axetil tablet cores of Examples 1 and 2 (- ◆ -: Tablet core of Example 1 containing silicon dioxide, - ■ -: Tablet core of Example 2 without silicon dioxide).
Fig. 2 is a graph according to the result of Experiment 2 showing the dissolution rate of the cefuroxime axetil capsules in Examples 3 and 4 (- ◆ -: Capsule of Example 3 containing silicon dioxide, - ■ -: Capsule of Example 4 without silicon dioxide).
Fig. 3 is a graph according to the result of Experiment 4 showing the dissolution rate of cefuroxime axetil tablet in Example 5 (test preparation) compared with that of the product according to Korean Patent No. 73572 (- ■-: Test preparation, -◆ -: Comparative preparation).
Fig. 4 is a graph according to the result of Experiment 5 showing the dissolution rate after 45 minutes of cefuroxime axetil tablet in Example 5 compared with that of the commercial product (- □ -: Tablet of Example 5, - ■ -: Commercial Product).

### BEST MODE FOR CARRYING OUT THE INVENTION

Hereinafter, the invention will be illustrated in more detail.

In an aspect, the present invention provides a pharmaceutical composition which comprises (i) amorphous cefuroxime axetil as an active ingredient, and (ii) silicon dioxide or its hydrate as a micro-environmental pH adjustor around cefuroxime axetil and an anti-gelling agent for cefuroxime axetil, wherein the weight ratio of cefuroxime axetil and silicon dioxide or its hydrate is 1:0.1 to 1:1.

The-present invention, in an another aspect, provides the above composition in an oral formulation such as tablet, capsule, granule, powder and dry syrup.

The composition according to the present invention, especially in the oral formulation such as tablet, capsule, granule, powder and dry syrup does not raise any problems involved in the prior art such as, for example, the degradation or gel formation of the cefuroxime axetil due to the absorption of moisture in air and the gel formation of cefuroxime axetil by the gastric juice upon administration and make it possible to increase the dissolution rate upon administration into patients, thereby improving the bioavailability.

As for an excipient to be suitably used in inhibiting the gelation of a drug which is susceptible to the gelation, it is important that the attractive force between each of the particles of the drug should be minimized and the particles of drug should be evenly dispersed into the dispersion medium. Hitherto, it was general to admix the drug with a disintegrant which can be well combined with the drug and has large disintegrating power to disperse the particles. However, if the conventional disintegrants are used, the gel formation of cefuroxime axetil by the absorption of moisture in air was not effectively prevented.

The composition according to the present invention contains silicon dioxide or its hydrate as a micro-environmental pH adjustor and an anti-gelling agent for cefuroxime axetil. According to the experiments by the present inventors, it was found that the hydrate form of silicon dioxide also showed the similar effect to silicon dioxide. Therefore, it is understood that even unless specifically indicated, the hydrate form of silicon dioxide is encompassed into silicon dioxide.

Silicon dioxide is an excipient which can be commonly used in the medicinal composition as a flow aid or a suspending agent due to its excellent fluidity and dispersity. The amount of the disintegrant which is generally used in the drug formulation in the art is within a range between 0.1% and 0.5% by weight. The excipient is also used as an adsorbent due to its broad surface area compared with other excipients.

Hitherto, silicon dioxide or its hydrate has not been used for the purpose of preventing gel formation in the cefuroxime axetil composition. As an example, the prevention of gel formation in the cefuroxime axetil film-coated tablet disclosed in KR Patent No. 73572 is made by disintegrant which is generally used in the art. In the above patent, silicon dioxide is used as lubricants and flow aids. Further, the trace amount of less than about 0.25 % by weight was used for the tablet. Therefore, it is apparent that the use of silicon dioxide is remarkably differentiated from the KR Patent No.73572 in aspects of the purpose and the mixing ratio, and thus, the KR patent never teaches the use of silicon dioxide as a anti-gelling agent for the cefuroxime axetil composition.

The mixing ratio of cefuroxime axetil with silicon dioxide or its hydrate used in the present invention in order to prevent gelation of cefuroxime axetil is not limited to a specific ratio, but is preferable to be used within the ratio between 1:0.1 - 1:1 by weight, with the ratio between 1:0.25 - 1:0.35 by weight being most preferable. If the amount of silicon dioxide is below 1:0.1 weight ratio, the gel formation of cefuroxime axetil cannot be sufficiently prevented. If the amount is larger than 1:1 weight ratio, a problem in the preparation process such as a difficulty in the tabletting process may arise.

The mixing of cefuroxime axetil and silicon dioxide or its hydrate is carried out in a conventional manner well known in the art with the conventional mixer.

Since silicon dioxide or its hydrate has an excellent fluidity, a high miscibility with cefuroxime axetil can be obtained. Even if relatively small amount of silicon dioxide is mixed with cefuroxime axetil, the particles of silicon dioxide are evenly distributed between the particles of cefuroxime axetil due to the broad surface area of silicon dioxide, thereby the attractive force between particles of cefuroxime axetil being reduced to eliminate electric charge and the occurrence of gelation of cefuroxime axetil being subsequently prevented.

Therefore, when an oral formulation prepared from the composition comprising a specific amount of silicon dioxide or its hydrate and an effective amount of cefuroxime axetil is administered into a patient, sufficient dissolution is made since the gel formation of cefuroxime axetil does not occur even if the rupture of a film is not rapidly proceeded, and in addition, gel formation is not occur even if moisture in air is absorbed into the formulation upon storage.

In addition, cefuroxime axetil reveals the lowest hydrolysis rate in the pH range between 3.5 and 5.5 (See, Pharmaceutical Research, Vol. 8, No. 7, 1991, p896).
In pH > 5.5, the decomposition rate of cefuroxime axetil increases (See, the Japanese journal of "Antibiotics & Chemotherapy", Vol. 7, No. 11, 1991). Therefore, when considering the stability of cefuroxime axetil in the micro-environment within the preparation, it is preferable that aqueous pH of the excipient be preferably a range between 3.5 and 5.5. Since a 4% aqueous suspension of silicon dioxide or its hydrate has pH values between 3.5 and 4.4, if cefuroxime axetil is mixed with silicon dioxide to prepare solid formulation for oral administration, the micro-environment around cefuroxime axetil is maintained with an acidic state between 3.5 and 5.5 to increase the stability of cefuroxime axetil. Therefore, silicon dioxide or its hydrate meets the acidic state requirement of tablet core of cefuroxime axetil.

The amount of silicon dioxide or its hydrate used in the composition of the invention is preferably 8 to 45% by weight based on the solid formulation. It is more preferable to use silicon dioxide or its hydrate in an amount of 10 to 20% by weight.

Cefuroxime axetil in the composition of the present invention is preferably used in an amount within the range from 50 mg to 500 mg based on cefuroxime per unit dose. Dose of cefuroxime axetil for adults will typically be 100 to 3,000 mg/day as cefuroxime and 125 to 1,000 mg/day for children by dividing it into two or more times per day, if necessary, although the precise dose may depend on the conditions of disease to be treated, the age and the severity of a patient and the frequency of administration.

The composition according to the present invention may be prepared in the oral formulation with the conventional pharmaceutical excipients, such as, for example, a disintegrant, a binder, a colorant, a solubilizer, a sweetener, a flavor, etc. Such oral formulation includes tablet, capsule, granule, powder and dry syrup, etc.

When cefuroxime axetil containing composition is prepared in a tablet formulation, it is preferable that the mixing ratio of cefuroxime axetil and silicon dioxide or its hydrate is within a range from 1:0.1 to 1:1 by weight. The composition may be contained in an amount of 16 to 90% by weight based on the tablet, with the range from 20 to 80% by weight being more preferable. It is preferable that a unit tablet contain cefuroxime axetil in an amount of the range from 50 to 500 mg.

A disintegrating agent may be added to the tablet in order to increase the dissolution rate by increasing the disintegrating rate of the tablet core of cefuroxime axetil. Examples of the excipient which may be desirably added include sodium starch glycolate, polacrilin potassium, low-substituted hydroxypropyl cellulose, etc.

The term, "low-substituted hydroxypropyl cellulose" used herein is defined as cellulose which contains not less than 5.0 percent and not more than 16 percent of hydroxy-propoxy group (-OCH₂CHOHCH₃).

In addition, the surface of tablet according to the invention may be film-coated with a film forming material in order to mask the bitter taste and to prevent moisture from penetrating into the tablet core.

In the present invention, as the film forming material, a polymeric material can be used without limitation, provided that it has a tight molecular structure and preferably has a molecular weight above 20,000 and more preferably can not be easily dissolved in an aqueous solution. The term "film coating material" or "film forming material" in the present invention can be interchangeably used with a term, "film substrate".

Furthermore, when considering the decomposition rate of cefuroxime axetil according to pH, it is most preferable for the film forming material to have acidic nature in an aqueous solution. That is, it is possible to enhance the stability of cefuroxime axetil by film-coating the surrounding of the tablet core with a polymeric material having an acidic nature to make pH of the micro-environment of cefaroxime axetil near 3.5 - 5.5 wherein the decomposition rate of cefuroxime axetil is the lowest. The film substrate used in the prior art, Korean Patent No. 73572, is a polymeric material of a neutral nature in an aqueous solution. However, this may provoke a serious problem to the stability of active ingredient by making the pH of the micro-environment of cefuroxime axetil neutral or above neutral.

Acid polymer which can be used as the film substrate in the present invention includes such as, for example, methacrylic copolymer (i.e., Eudragit L, Eudragit S, etc.), hydroxy propylmethylcellulose phthalate, cellulose acetate phthalate, hydroxypropylmethyl cellulose acetate succinate, polyvinyl alcohol, etc. The polymeric material can be used in combination of two or more materials based on the nature of each material. This may be used in combination with other water soluble polymer materials in the film coating of the tablet core of cefuroxime axetil.

The film forming material may contain one or more excipients which are conventionally added to the film substrate, examples of which are a plasticizer, a preservatives, a colorant, a sunscreen, etc. These excipients make the coating process smooth and improve the nature and shape of the film. The amount to apply the film forming material may vary depending upon the nature of each film substrate and can be determined by taking into consideration the stability and the dissolution rate of cefuroxime axetil. The thickness of the film may be various since the tablet according to the present invention shows good dissolution rate regardless of the rupture rate of film.

It is also possible to present cefuroxime axetil-containing composition in the form of a capsule.

When cefuroxime axetil containing composition is prepared in a capsule formulation, it is preferable that the mixing ratio of cefuroxime axetil and silicon dioxide or its hydrate is 1:0.1 to 1:1 by weight. The composition may be contained in an amount of 16 to 90% by weight based on the capsule, with the range from 20 to 80% by weight being more preferable. It is preferable that a unit capsule contain cefuroxime axetil in the range from 50 to 500 mg as cefuroxime.

Other excipients may be added into the capsule formulation. A disintegrant may be added to the capsule in order to increase the dissolution rate by increasing the disintegrating rate of the capsule. Examples of the excipient which may be desirably added include sodium starch glycolate, polacrilin potassium, low-substituted hydroxypropyl cellulose, etc. The excipient may be used alone or in combination.

In addition, the composition according to the present invention may be prepared in the form of a granule, a powder or a dry syrup by masking the bitter taste by coating it with a water insoluble material or by adding an appropriate material. These formulations are also encompassed by the formulation of the present invention, but the present invention is not limited thereto.

### EXAMPLES

The present invention will be described in greater detail through the following examples. The examples are presented for illustrating purposes only and should not be construed as limiting the invention which is properly delineated in the claims.

### Example 1:

The following components were blended together, and compacted into granules with a roller compactor. The resulting granules were then compressed with a conventional tabletting machine.

| Components | Contents (mg/tablet) |
|---|---|
| Cefuroxime axetil | the amount equivalent to 250.00 mg of Cefuroxime |
| Silicon dioxide | 80.0 |
| Low substituted hydroxypropyl cellulose | 40.0 |
| Sodium starch glycolate | 150.0 |
| Stearic acid | 30.0 |

### Example 2

The following components were blended together, and compacted into granules with a roller compactor. The resulting granules were then compressed with a conventional tabletting machine.

| Components | Contents (mg/tablet) |
|---|---|
| Cefuroxime axetil | the amount equivalent to 250.00 mg of Cefuroxime |
| Low substituted hydroxypropyl cellulose | 40.0 |
| Sodium starch glycolate | 150.0 |
| Stearic acid | 30.0 |

### Example 3

### 1. Capsule contents

The following components were blended together, and compacted into granules with a roller compactor. The resulting granules were then filled into a hard capsule with a conventional capsule filler.

| Components | Contents (mg/capsule) |
|---|---|
| Cefuroxime axetil | the amount equivalent to 250.00 mg of Cefuroxime |
| Silicon dioxide | 300.0 |
| Low substituted hydroxypropyl cellulose | 80.0 |
| Sodium starch glycolate | 110.0 |
| Stearic acid | 30.0 |

### Example 4

### 1. Capsule contents

The following components were blended together, and compacted into granules with a roller compactor. The resulting granules were then filled into a hard capsule with a conventional capsule filler.

| Components | Contents (mg/capsule) |
|---|---|
| Cefuroxime axetil | the amount equivalent to 250.00 mg of Cefuroxime |
| Low substituted hydroxypropyl cellulose | 80.0 |
| Sodium starch glycolate | 110.0 |
| Stearic acid | 30.0 |

### Example 5

### 1. Tablet core

The following components were blended together, and compacted into granules with a roller compactor. The resulting granules were then compressed with a conventional tabletting machine.

| Components | Contents (mg/tablet) |
|---|---|
| Cefuroxime axetil | the amount equivalent to 250.00 mg of Cefuroxime |
| Silicon dioxide | 80.0 |
| Low substituted hydroxypropyl cellulose | 80.0 |
| Sodium starch glycolate | 110.0 |
| Stearic acid | 30.0 |

### 2. Film forming composition

The tablet cores as prepared in the above step were applied to a conventional pan coater and the film forming material having the following composition was sprayed in a conventional manner to coat the tablets. The amount of the coating material was adjusted to a range between 0.005 and 0.05 parts by weight based on the weight of the tablet core.

| Components | Added amount (%) |
|---|---|
| Eudragit L 30 D | 16.6 |
| Talc | 4.0 |
| Polyethylene glycol 6000 | 1.6 |
| Purified water up to | 100.0 |

### Examples 6 to 14

Tablet cores and film forming compositions of Examples 6 to 14 were prepared according to the same manner as in Example 5 except for the components specified in each example.

### Example 6

### 1. Tablet core

| Components | Contents (mg/tablet) |
|---|---|
| Cefuroxime axetil | the amount equivalent to 250.00 mg of Cefuroxime |
| Silicon dioxide | 35.0 |
| Low substituted hydroxypropyl cellulose | 80.0 |
| Sodium starch glycolate | 155.0 |
| Polyethylene glycol 6000 | 30.0 |

### 2. Film forming composition

| Components | Added amount (%) |
|---|---|
| Eudragit L 30 D | 16.6 |
| Talc | 4.0 |
| Polyethylene glycol 6000 | 1.6 |
| Purified water up to | 100.0 |

### Example 7

### 1. Tablet core

| Components | Contents (mg/tablet) |
|---|---|
| Cefuroxime axetil | the amount equivalent to 250.00 mg of Cefuroxime |
| Silicon dioxide | 80.0 |
| Low substituted hydroxypropyl cellulose | 70.0 |
| Polacrilin potassium | 110.0 |
| Stearic acid | 30.0 |

### 2. Film forming composition

| Components | Added amount (%) |
|---|---|
| Eudragit L 30 D | 16.6 |
| Talc | 4.0 |
| Purified water up to | 100.0 |

### Example 8

### 1. Tablet core

| Components | Contents (mg/tablet) |
|---|---|
| Cefuroxime axetil | the amount equivalent to 250.00 mg of Cefuroxime |
| Silicon dioxide | 105.0 |
| Low substituted hydroxypropyl cellulose | 70.0 |
| Sodium starch glycolate | 75.0 |
| Polyethylene glycol 6000 | 20.0 |

### 2. Film forming composition

| Components | Added amount (%) |
|---|---|
| Eudragit L 30 D | 6.0 |
| Talc | 3.0 |
| Triethyl citrate | 0.6 |
| Purified water | 5.0 |
| Isopropyl alcohol up to | 100.0 |

### Example 9

### 1. Tablet core

| Components | Contents (mg/tablet) |
|---|---|
| Cefuroxime axetil | the amount equivalent to 250.00 mg of Cefuroxime |
| Silicon dioxide monohydrate | 125.0 |
| Low substituted hydroxypropyl cellulose | 90.0 |
| Sodium starch glycolate | 60.0 |
| Magnesium Stearate | 30.0 |

### 2. Film forming composition

| Components | Added amount (%) |
|---|---|
| Hydroxypropylmethylcellulose phthalate | 5.0 |
| Triacetin | 3.0 |
| Hydroxypropylmethyl cellulose | 0.02 |
| Purified water up to | 100.0 |

### Example 10

### 1. Tablet core

| Components | Contents (mg/tablet) |
|---|---|
| Cefuroxime axetil | the amount equivalent to 500.00 mg of Cefuroxime |
| Silicon dioxide monohydrate | 210.0 |
| Low substituted hydroxypropyl cellulose | 190.0 |
| Sodium starch glycolate | 150.0 |
| Stearic acid | 30.0 |

### 2. Film forming composition

| Components | Added amount (%) |
|---|---|
| Eudragit L 30 D | 16.6 |
| Talc | 4.0 |
| Triacetin | 1.6 |
| Hydroxypropylmethyl cellulose | 0.02 |
| Purified water up to | 100.0 |

### Example 11

### 1. Tablet core

| Components | Contents (mg/tablet) |
|---|---|
| Cefuroxime axetil | the amount equivalent to 500.00 mg of Cefuroxime |
| Silicon dioxide | 160.0 |
| Low substituted hydroxypropyl cellulose | 160.0 |
| Sodium starch glycolate | 220.0 |
| Stearic acid | 50.0 |

### 2. Film forming composition

| Components | Added amount (%) |
|---|---|
| Hydroxypropylmethylcellulose phthalate | 5.0 |
| Triacetin | 3.0 |
| Hydroxypropylmethyl cellulose | 0.02 |
| Purified water up to | 100.0 |

### Example 12

### 1. Tablet core

| Components | Contents (mg/tablet) |
|---|---|
| Cefuroxime axetil | the amount equivalent to 125.00 mg of Cefuroxime |
| Silicon dioxide | 125.0 |
| Low substituted hydroxypropyl cellulose | 100.0 |
| Sodium starch glycolate | 90.0 |
| Stearic acid | 15.0 |

### 2. Film forming composition

| Components | Added amount (%) |
|---|---|
| Eudragit L 30 D | 16.6 |
| Talc | 4.0 |
| Triacetin | 1.6 |
| Hydroxypropylmethyl cellulose | 0.02 |
| Purified water up to | 100.0 |

### Example 13

### 1. Tablet core

| Components | Contents (mg/tablet) |
|---|---|
| Cefuroxime axetil | the amount equivalent to 125.00 mg of Cefuroxime |
| Silicon dioxide | 40.0 |
| Low substituted hydroxypropyl cellulose | 40.0 |
| Sodium starch glycolate | 60.0 |
| Stearic acid | 15.0 |

### 2. Film forming composition

| Components | Added amount (%) |
|---|---|
| Eudragit L 30 D | 16.6 |
| Talc | 4.0 |
| Triacetin | 1.6 |
| Hydroxypropylmethyl cellulose | 0.02 |
| Purified water up to | 100.0 |

### Example 14

### 1. Tablet core

| Components | Contents (mg/tablet) |
|---|---|
| Cefuroxime axetil | the amount equivalent to 125.00 mg of Cefuroxime |
| Silicon dioxide monohydrate | 53.0 |
| Low substituted hydroxypropyl cellulose | 45.0 |
| Sodium starch glycolate | 50.0 |
| Magnesium stearate | 10.0 |

### 2. Film forming composition

| Components | Added amount (%) |
|---|---|
| Hydroxypropylmethylcellulose phthalate | 5.0 |
| Triacetin | 3.0 |
| Hydroxypropylmethyl cellulose | 0.02 |
| Purified water up to | 100.0 |

### Experiment 1

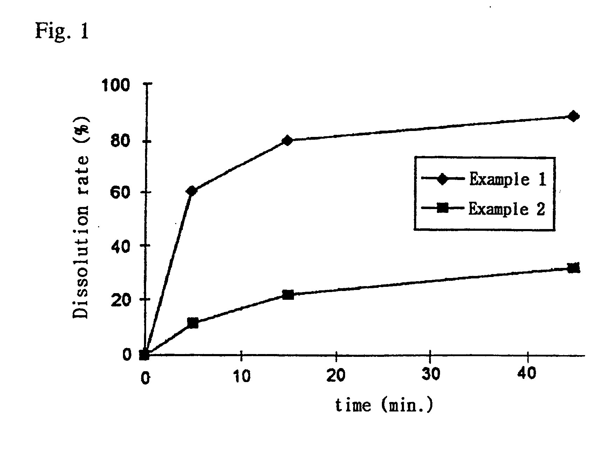

In order to demonstrate the effect that silicon dioxide prevents cefuroxime axetil from being gelled, a dissolution test was conducted for the tablets according to Examples 1 and 2 in accordance with the method illustrated in the monograph of the cefuroxime axetil tablets of Vol. 23, on page 316 of the U.S. pharmacopeia. The results are shown in Fig. 1.

As can be seen from the Fig. 1, the tablet of Example 1 containing 80 mg of silicon dioxide showed 87.2% of dissolution rate after 45 minutes while that of Example 2 which is devoid of silicon dioxide showed only 30.4% of dissolution rate and left undissolved small mass in dissolution medium. Therefore, it is noted that silicon dioxide is excellent in preventing gel formation of cefuroxime axetil.

### Experiment 2

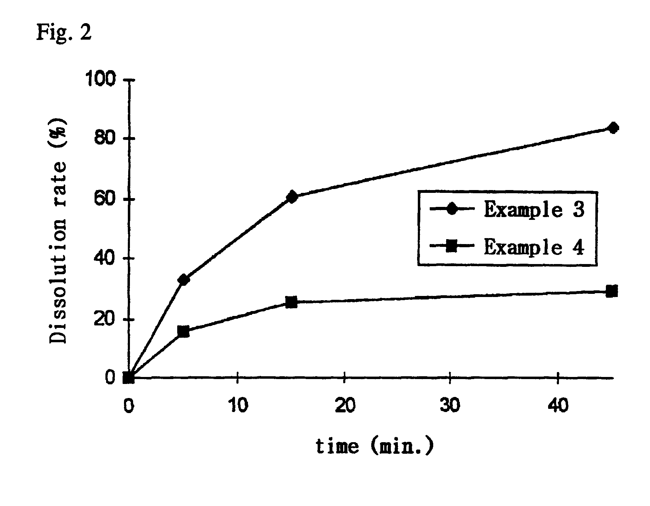

In order to demonstrate the effect that silicon dioxide prevents cefuroxime axetil from being gelled in the capsule preparation, a dissolution test was conducted for the capsules according to Examples 3 and 4 in accordance with the method illustrated in the monograph of the cefuroxime axetil tablets of Vol. 23, on page 316 of the U.S. pharmacopeia. The results are shown in Fig. 2.

As can be seen from the Fig. 2, the capsule of Example 3 containing 300 mg of silicon dioxide showed 84.3 % of dissolution rate after 45 minutes while that of Example 4 which is devoid of silicon dioxide showed only 29.6% of dissolution rate. Therefore, it is noted that silicon dioxide in the capsule preparation is excellent in preventing gel formation of cefuroxime axetil.

### Experiment 3

In order to determine the effect that silicon dioxide stabilize cefuroxime axetil, a tablet core of Example 5 was stored under the accelerated test condition. The test was carried out with naked tablets in order to exclude any effect of the film forming material on the stability of the tablet. The storage condition in the accelerated test was 40 °C and 75% of relative humidity. Upon storage of the tablet in open state for 30 days, the moisture content and dissolution rate were determined. The results are shown in Table 1.

**Table 1**

| | Tablet core of Example 5 | |
|---|---|---|
| Storage time(day) | 0 | 30 |
| Moisture contents | 1.21 % | 3.03% |
| Content | 99.39% | 97.81% |

As can be seen from Table 1, it was found that even if the tablet core according to Example 5 was stored under the accelerated condition in the naked and opened state, the moisture content after 30 days was increased to 3.3% while only 1.58% of the content was changed (from 99.39 % to 97.81%). Therefore, it is noted that the composition of the tablet core according to Example 5 has excellent stability even under the severe condition.

### Experiment 4

In order to compare the tablet according to the present invention with that of the prior art, the tablet of the present intention in which a tablet core comprising effective amount of cefuroxime axetil and a specific amount of silicon dioxide was coated with the acidic film forming material (test preparation) and that of Example 3 of Korean Patent No. 73572 (comparative preparation) were subjected to dissolution test in accordance with the method illustrated in the monograph of the cefuroxime axetil tablets of Vol. 23, on page 316 of the U.S. pharmacopeia. The test preparation was the tablet according to Example 5 of the present invention and the comparative preparation was prepared as follows:

The tablet core of the comparative preparation was prepared according to Example 3 of Korean Patent No. 73572, except that titanium dioxide was used in stead of Opaspray Blue M-1-4395B in the film forming composition in Example 3. The components employed were set forth in the following tables. Opaspray, which is based on titanium dioxide with lake colors, is presumed to give a sunscreen effect and to reveal a color. This pigment is known to commercially available from Colorcon Ltd. at Orpington of Kent, United Kingdom. However, since it was difficult to purchase the product and to find out the exact composition thereof, the inventors used titanium dioxide. When titanium dioxide was only added into the film forming composition in lieu of opaspray blue M-1-4395B, it was observed that the rupture time of the film in the solution of 0.07 M of hydrochloric acid was below 40 seconds, exactly less than 10 seconds. The rupture time was measured as the time which elapses before the core of the tablet first become visible to the naked eye through the ruptured film coat. Therefore, it was confirmed that the substitution of the pigment did not influence any effect on the dissolution rate of the tablet according to Example 3 of Korean Patent No. 73572.

### 1. Tablet core

The following components except for silicon dioxide were blended together, and compacted with a roller compactor. The compacted materials were comminuted with an oscillating granulator and the resulting granules were blended with silicon dioxide, and were then the compressed with a conventional tableting machine.

| Components | Contents (mg/tablet) |
|---|---|
| Cefuroxime axetil | the amount equivalent to 250.00 mg of Cefuroxime |
| Microcrystalline cellulose | 94.55 |
| Croscarmellose sodium Type A | 15.50 |
| Sodium lauryl sulphate | 4.50 |
| Silicon dioxide | 1.25 |
| Hydrogenated vegetable oil | 8.50 |

### 2. Film forming composition

The tablet cores as prepared in the above step were put into a conventional pan coater and the film forming material having the following composition was sprayed in the conventional manner to coat the tablets. The tablet was coated with a target coat weight of 1 mg per 32 mm².

| Components | Added amount (%) |
|---|---|
| Hydroxypropylmethylcellulose 6 | 10.0 |
| Propylene glycol | 0.60 |
| Methyl hydroxybenzoate | 0.10 |
| Propyl hydroxybenzoate | 0.08 |
| Titanium dioxide | 10.00 |
| Purified water up to | 100.0 |

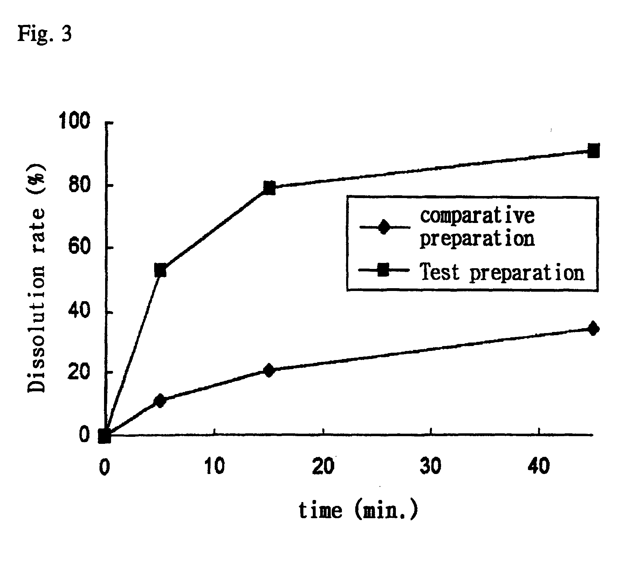

The dissolution rate results were set forth in figure 3. As can be seen from the Fig. 3, the test preparation according to the invention showed 91% of dissolution rate after 45 minutes while the comparative preparation showed only 35%. Therefore, it was found that the test preparation is superior to the comparative preparation in the dissolution rate.

### Experiment 5

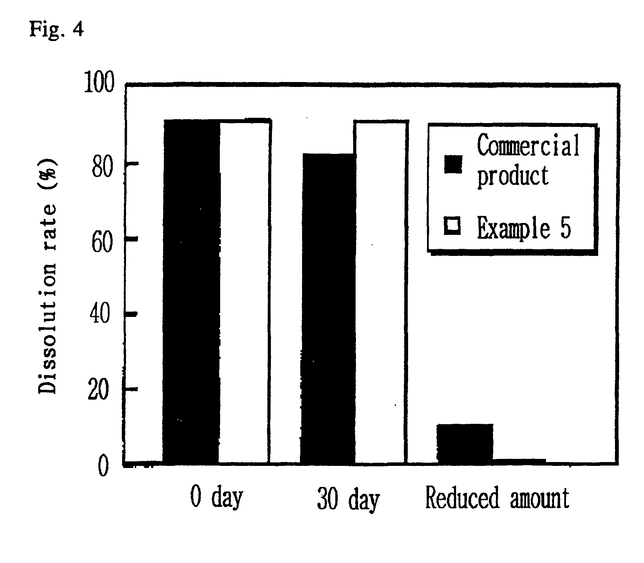

Each 250 mg of the coated tablet of Example 5 and the commercial cefuroxime axetil tablet (available from Glaxo Wellcome P.L.C.) were stored under the accelerated test condition and subjected to the content test and dissolution rate test in accordance with the method illustrated in the monograph of the cefuroxime axetil tablets of vol. 23, on page 316 of the U.S. Pharmacopeia. The storage condition in the accelerated test was 40 °C and 75% of relative humidity. After storing the coated tablet of Example 5 and the commercial product under the above condition in an opened state for 30 days, the content change and dissolution rate test were compared. The results are shown in Fig. 4 and Tables 2 and 3.

**Table 2**

| | | Commercial product | | Tablet of Example 5 | |
|---|---|---|---|---|---|
| Storage time(day) | | 0 | 30 | 0 | 30 |
| Dissolution rate | 15 min. | 66.77 | 66.47 | 78.86 | 75.76 |
| | 45 min. | 91.16 | 81.94 | 91.20 | 90.84 |
| Reduced amount* | | 9.22 | | 0.36 | |

| | | | | | |
|---|---|---|---|---|---|
| Reduced amount*: Reduced amount of the drug dissolved at 45 min. during the storage period(0-30 days) | | | | | |

**Table 3**

| | Commercial product | | Tablet of Example 5 | |
|---|---|---|---|---|
| Storage time(day) | 0 | 30 | 0 | 30 |
| Contents | 99.65 | 97.63 | 99.78 | 99.45 |
| Decreased amount* | 2.02 | | 0.33 | |

| | | | | |
|---|---|---|---|---|
| Decreased amount*: Decreased amount of content in the product during the storage period | | | | |

As can be seen from the Fig. 4 and the above Tables 2 and 3 which reveal the changes in contents and dissolution rates before and 30 days after storage under the accelerated condition, the dissolution rate of the commercial product at 45 minute was decreased by 9.22% after storing 30 days while that of the present invention (Example 5) was decreased by only 0.36%. As can be seen from Table 3, the contents of the cefuroxime axetil in the commercial product after 30 days was decreased by 2.02% while that of the present invention was decreased by only 0.33%. From the above results, the coated tablet according to the present invention shows excellent stability in the dissolution rate and the contents, and thus, the tablet according to the invention is much more stable than the commercial product.

### INDUSTRIAL APPLICABILITY

As can be seen from the above Experiments 1 to 5, the composition according to the present invention is characterized in that it comprises cefuroxime axetil and silicon dioxide or its hydrate as a micro-environmental pH adjustor and an anti-gelling agent for cefuroxime axetil can prevent the degradation or gel formation of the cefuroxime axetil due to the absorption of moisture in air upon long term storage and make it possible to increase the dissolution rate of cefuroxime axetil regardless of the disintegration rate of the preparation containing cefuroxime axetil, thereby improving the bioavailability. In Korean Patent No. 73572, it is only possible to avoid gel formation of cefuroxime axetil by providing specific tablet comprising a film which should be rapidly ruptured in an aqueous hydrochloric acid solution and a tablet core which is disintegrated immediately after the film is ruptured. However, according to the present invention, the gel formation of cefuroxime axetil in oral formulation in the form of tablet, capsule, granule, powder and dry syrup is prevented and an excellent dissolution rete is obtained.

## Claims

1. A pharmaceutical composition which comprises (i) amorphous cefuroxime axetil as an active ingredient, and (ii) silicon dioxide or its hydrate as a micro-environmental pH adjustor around cefuroxime axetil and an anti-gelling agent for cefuroxime axetil; wherein the weight ratio of cefuroxime axetil and silicon dioxide or its hydrate is 1:0.1 to 1:1.

2. The composition of Claim 1, wherein the weight ratio of cefuroxime axetil and silicon dioxide or its hydrate is 1:0.25 to 1:0.35.

3. The composition of Claim 1 or 2 in the form of an oral formulation selected from the group consisting of tablets, capsules, granules, powders and dry syrups.

4. The composition of Claim 3 in the form of tablet or capsule formulation wherein cefuroxime axetil and silicon dioxide or its hydrate are contained in an amount of 16 to 90 % by weight based on the total amount of the formulation.

5. The composition of Claim 4 in the form of tablet wherein a tablet core containing amorphous cefuroxime axetil as an active ingredient, silicon dioxide or its hydrate and pharmaceutically acceptable excipients is film-coated with the film forming material comprising acidic polymeric material(s).

6. The composition of Claim 5, wherein the acidic polymeric material is selected from the group consisting of methacrylic copolymer, hydroxypropylmethylcellulose phthalate, celluloseacetate phthalate and hydroxypropylmethylcellulose acetate succinate, and a mixture thereof.

## Patentansprüche

1. Pharmazeutische Zusammensetzung umfassend (i) amorphes Cefuroximaxetil als einen Wirkstoff, und (ii) Siliziumdioxid oder sein Hydrat als einen pH-Regulierer der Mikroumgebung um Cefuroximaxetil und ein Anti-Geliermittel für Cefuroximaxetil; wobei das Gewichtsverhältnis von Cefuroximaxetil und Siliziumdioxid oder seinem Hydrat 1:0,1 bis 1:1 ist.

2. Zusammensetzung nach Anspruch 1, wobei das Gewichtsverhältnis von Cefuroximaxetil und Siliziumdioxid oder seinem Hydrat 1:0,25 bis 1:0,35 ist.

3. Zusammensetzung nach Anspruch 1 oder 2 in Form einer oralen Formulierung ausgewählt aus der Gruppe bestehend aus Tabletten, Kapseln, Körnchen, Pulvern und Trockensirupe.

4. Zusammensetzung nach Anspruch 3 in Form einer Tabletten- oder Kapselformulierung, wobei Cefuroximaxetil und Siliziumdioxid oder sein Hydrat in einer Menge von 16 bis 90 Gew%, basierend auf der Gesamtmenge der Formulierung, enthalten sind.

5. Zusammensetzung nach Anspruch 4 in Form einer Tablette, wobei ein Tablettenkern enthaltend amorphes Cefuroximaxetil als einen Wirkstoff, Siliziumdioxid oder sein Hydrat und pharmazeutisch annehmbare Arzneimittelträger mit einem Film beschichtet ist, wobei das den Film bildende Material saure(s) Polymermaterial(ien) umfasst.

6. Zusammensetzung nach Anspruch 5, wobei das saure Polymermaterial ausgewählt ist aus der Gruppe bestehend aus Methacrylcopolymer, Hydroxypropylmethylzellulosephthalat, Zelluloseacetatphthalat und Hydroxypropylmethylzelluloseacetatsuccinat, und ein Gemisch davon.

## Revendications

1. Une composition pharmaceutique qui comprend (i) du céfuroxime axetil comme principe actif, et (ii) du dioxyde de silicium ou son hydrate comme agent d'ajustement du pH micro-environnemental autour du céfuroxime axetil et un agent anti-gélifiant pour le céfuroxime axetil, dans laquelle le rapport pondérai du céfuroxime axetil et du dioxyde de silicium ou son hydrate est de 1:0,1 à 1:1.

2. La composition de la revendication 1, dans laquelle le rapport pondéral du céfuroxime axetil et du dioxyde de silicium ou son hydrate est de 1:0,25 à 1:0,35.

3. La composition de la revendication 1 ou 2, sous forme de formulation orale choisie dans le groupe constitué par les comprimés, les capsules, les granules, les poudres et les sirops secs.

4. La composition de la revendication 3 sous forme de formulation comprimé ou capsule, dans laquelle le céfuroxime axetil et le dioxyde de silicium ou son hydrate sont présents à une teneur de 16 à 90 % en poids par rapport à la quantité totale de la formulation.

5. La composition de la revendication 4 sous forme de comprimé, dans laquelle un noyau de comprimé contenant du céfuroxime axetil comme principe actif, du dioxyde de silicium ou son hydrate et des excipients pharmaceutiquement acceptables est pelliculé, avec le matériau formant le film comprenant un (des) matériau(x) polymère(s) acide(s).

6. La composition de la revendication 5, dans laquelle le matériau polymère acide est choisi dans le groupe constitué par un copolymère méthacrylique, le phtalate d'hydroxypropylméthylcellulose, le phtalate d'acétate de cellulose et le phtalate d'hydroxypropylméthylcellulose, et un de leurs mélanges.
